# EUROPÄISCHE PATENTSCHRIFT

(11) **EP 0 609 667 B1**
(45) Veröffentlichungstag und Bekanntmachung des Hinweises auf die Patenterteilung: **08.01.2003**
(21) Anmeldenummer: 94100365.9
(22) Anmeldetag: 12.01.1994
(51) Int. Cl.: A61L 31/00

(54) **Abdeckmembran**
Covering membrane
Membrane de recouvrement

(30) Priorität: 01.02.1993 DE 4302708
(43) Veröffentlichungstag der Anmeldung: 10.08.1994
(73) Patentinhaber: Kirsch, Axel, Dr., 70794 Filderstadt (DE)
(72) Erfinder: Kirsch, Axel, Dr., 70794 Filderstadt (DE)
(74) Vertreter: Goddar, Heinz J., Dr.

(56) Entgegenhaltungen:
- EP-A- 0 213 563
- WO-A-90/13302
- WO-A-92/10218
- WO-A-93/02718
- WO-A-93/24097
- DE-A- 3 607 075
- FR-A- 2 664 501

## Beschreibung

Die Erfindung betrifft eine Abdeckmembran zum vorübergehenden Abdecken einer mit Knochenaufbaumaterial, wie Hydroxylapatitgranulat, gefüllten Ausnehmung in körpereigenem Knochengewebe.

In der Knochenchirugie, beispielsweise bei der Rekonstruktion von Knochen in der plastischen Chirugie oder bei kieferchirugischen Operationen, ist es üblich, Knochendefektstellen in Form von Ausnehmungen oder Höhlungen im körpereigenen Knochengewebe mit Knochenaufbaumaterial zu füllen, welches in der Regel aus einer Mischung aus Knochenersatzmaterial, wie Hydroxylapatitgranulat, und körpereigenen Knochenpartikeln besteht. Um zu gewährleisten, daß das Knochenaufbaumaterial im wesentlichen ausschließlich von der Knochenseite her knöchern durchwachsen wird, nicht aber in unerwünschter Weise von Schleimhautgewebe, wird die Ausnehmung mit einer Abdeckmembran der eingangs genannten Art verschlossen, die verhindern soll, daß ein Durchwachsen des Knochenaufbaumaterials mit Nicht-Knochengewebe erfolgt. Nur dann nämlich, wenn ein vollständig knöchernes Durchwachsen des Knochenaufbaumaterials gewährleistet ist, läßt sich die Knochendefektstelle im wesentlichen vollständig beseitigen und das Knochenaufbaumaterial nach knöchernem Durchwachsen in den körpereigenen Knochen reintegrieren.

Bislang finden als Abdeckmembranen beispielsweise Polytetrafluorethylenfolien Verwendung, die jedoch den Nachteil haben, daß sie nach Ausheilen der Knochendefektstelle im Körper verbleiben und hierdurch zu Komplikationen Anlaß geben können.

WO 90/13302 offenbart eine Abdeckmembran zur Behandlung von Knochendefekten, die aus einem resorbierbaren Material besteht. Die Membran ist an mindestens einer Seite mit bioaktiven Substanzen, wie antimikrobiellen Substanzen, Proteinen oder dergleichen versehen. Um an bestimmten Stellen auf einer Seite einer Membran die bioaktiven Substanzen zu unterschiedlichen Zeiten freizusetzen, sind auf der entsprechenden Seite eine oder mehrere Schichten aus einem resorbierbaren Material aufgebracht, welche die Bereiche mit den bioaktiven Substanzen überdecken. Dabei kann vorgesehen sein, daß in einer solchen Schicht an verschiedenen Stellen die Resorptionsrate unterschiedlich ist.

WO 92/10218 offenbart eine Knochenabdeckmembran aus einem biologisch abbaubaren Material, welche aus einer Barrierenschicht, die das Durchtreten von Zellen verhindern soll, und einer Fasermatrix, in welche Gewebe einwachsen soll, besteht.

Der Erfindung liegt die Aufgabe zugrunde, eine Abdeckmembran der gattungsgemäßen Art zu schaffen, welche das Auftreten von Spätkomplikationen nach dem Ausheilen von Knochendefektstellen vermeidet.

Erfindungsgemäß wird diese Aufgabe dadurch gelöst, daß die Abdeckmembran aus resorbierbarem Membranmaterial besteht.

Dabei kann vorgesehen sein, daß das Membranmaterial von der dem körpereigenen Knochen abgewandten Seite schneller resorbierbar ist als von der dem körpereigenen Knochen zugewandten Seite.

Die Erfindung schlägt auch vor, daß die Dichte des Membranmaterials von der dem körpereigenen Knochen abgewandten Seite zu der dem körpereigenen Knochen zugewandten Seite zunimmt.

Nach der Erfindung kann auch vorgesehen sein, daß das Membranmaterial aus mehreren Schichten unterschiedlicher Dichte und/oder Zusammensetzung besteht.

Eine weitere Ausführungsform der Erfindung sieht dabei vor, daß das Membranmaterial mehrere gewebte oder gewirkte Schichten unterschiedlicher Textur aufweist.

Nach der Erfindung kann auch vorgesehen sein, daß das Membranmaterial zumindest teilweise aus lyophilisierter Hirnhaut (dura mater) besteht.

Die Erfindung schlägt auch vor, daß das Membranmaterial zumindest teilweise aus Folyglactid/vicryl besteht.

Auch kann vorgesehen sein, daß das Membranmaterial zumindest teilweise aus Kollagen besteht.

Die Erfindung kann ferner dadurch gekennzeichnet sein, daß das Membranmaterial zumindest teilweise aus Polylactid besteht.

Eine weitere Ausführungsform der Abdeckmembran nach der Erfindung ist dadurch gekennzeichnet, daß das Membranmaterial zumindest teilweise aus Oxymethylcellulose besteht.

Die Abdeckmembran nach der Erfindung kann in vorteilhafter Weise auch gekennzeichnet sein durch eine derartige Einstellung des von der dem körpereigenen Knochen abgewandten Seite zu dem körpereigenen Knochen zugewandten Seite abnehmenden Resorbierbarkeitsgradienten des Membranmaterials, daß die am Knochen anliegende Schicht des Membranmaterials erst dann von körpereigenem Gewebe resorbiert wird, wenn die durch die Abdeckmembran verschlossene Ausnehmung des körpereigenen Knochens im wesentlichen vollständig knöchern durchwachsen und in den Knochen reintegriert ist.

Auch ist die Erfindung gekennzeichnet durch eine Versteifungslage in Form eines perforierten Metallbleches oder dergleichen.

Dabei kann auch vorgesehen sein, daß das Metallblech aus Titan besteht.

Der Erfindung liegt die überraschende Erkenntnis zugrunde, daß es gelingt, die Nachteile der bislang bekannten, im Körper Nicht-abbau- oder resorbierbaren Abdeckmembranen dadurch zu vermeiden, daß ein Membranmaterial verwendet wird, welches im Körper resorbierbar ist. Besonders vorteilhaft ist es dabei, wenn, wie der Kerngedanke der Erfindung vorsieht, ein Membranmaterial verwendet wird, dessen Resorbierbarkeit von der dem körpereigenen Knochengewebe abgewandten Seite zu der dem körpereigenen Knochengewebe zugewandten Seite abnimmt. Hierdurch wird erreicht, daß die Abdeckmembran im wesentlichen vollständig von der dem Knochen abgewandten Seite her resorbiert wird, so daß also das knöcherne Durchwachsen des Knochenaufbaumaterials und damit das Ausheilen des Knochendefektes im wesentlichen ungestört von dem jedwede Resorption von körperfremdem Gewebe begleitenden Entzündungsprozeß erfolgen kann.

Wenn die Abdeckmembran eine Versteifungslage aufweist, die auch an der dem Knochen zu- oder abgewandten Seite der Abdeckmembran von dieser separat angeordnet sein kann, gelingt es, Beeinträchtigungen des Osteosyntheseprozesses durch Bewegungen der Abdeckmembran zu verhindern, so daß der Knochenaufbauprozeß besonders gefördert wird.

Weitere Merkmale und Vorteile der Erfindung ergeben sich aus der nachstehenden Beschreibung, in der ein Ausführungsbeispiel anhand der schematischen Zeichnung im einzelnen erläutert ist. Dabei zeigt:
- Fig. 1: eine mit einer Abdeckmembran nach einem Ausführungsbeispiel gemäß der Erfindung abgedeckte Knochendefektstelle im Schnitt senkrecht durch die Membranebene; und
- Fig. 2: die Abdeckmembran gemäß Figur 1 im Schnitt senkrecht zur Membranebene, stark vergrößert.

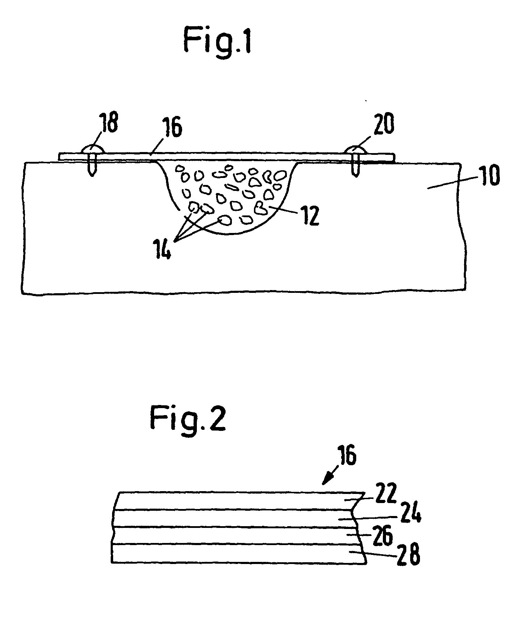

Wie Figur 1 erkennen läßt, ist dort eine in einem körpereigenen Knochen 10 durch eine Ausnehmung 12 gebildete Knochendefektstelle mit Knochenaufbaumaterial 14 aus Hydroxylapatitgranulat im wesentlichen vollständig gefüllt, wobei diesem Hydroxylapatitgranulat in bekannter Weise aus körpereigenem Knochengewebe bestehende Knochenpartikel beigemengt sind. Die mit dem Knochenaufbaumaterial 14 gefüllte Ausnehmung 12 ist von einer Abdeckmembran 16 abgedeckt, die allseits der Ausnehmung 12 mittels Befestigungsnägeln 18, 20 in dichter Anlage am körpereigenen Knochen 10 befestigt ist. Die Abdeckmembran 16 hat den Zweck, das knöcherne Durchwachsen des Knochenaufbaumaterials 14 vom körpereigenen Knochen 10 her in der Weise zu gewährleisten, daß das Durchwachsen des Knochenaufbaumaterials mit anderem als Knochengewebe, insbesondere Schleimhautgewebe, verhindert wird.

Die Abdeckmembran 18 besteht bei dem gezeigten Ausführungsbeispiel aus mehreren Schichten (Figur 2) 22, 24, 26, 28, wobei die Dichte der Schichten von der dem Knochen 10 in eingesetztem Zustand (Figur 1) abgewandten Seite zu der dem Knochen zugewandten Seite zunimmt. Dies ist dadurch bewerkstelligt, daß bei dem gezeigten Ausführungsbeispiel sämtliche Schichten 22 - 28 aus Kollagengewebe bestehen, also identische Zusammensetzung haben, wobei aber die Webdichte in der Schicht 22 verhältnismäßig gering, hingegen, bis dorthin schichtweise ansteigend, in der Schicht 28 sehr groß ist.

Durch die ansteigende Dichte der Abdeckmembran 16 von der Schicht 22 zur Schicht 28 hin wird gewährleistet, daß die Abdeckmembran 16 von der dem Knochen abgewandten Seite her resorbiert wird, d. h. also, die dem Knochen 10 zugewandte Schicht 28 wird erst ganz zuletzt resorbiert, nachdem die Ausnehmung 12 bzw. das darin befindliche Knochenaufbaumaterial 14 wieder im wesentlichen vollständig mit körpereigener Knochenmasse durchwachsen und damit in dem Knochen 10 integriert ist.

Die in der vorstehenden Beschreibung, in der Zeichnung sowie in den Ansprüchen offenbarten Merkmale der Erfindung können sowohl einzeln als auch in beliebigen Kombinationen für die Verwirklichung der Erfindung in ihren verschiedenen Ausführungsformen wesentlich sein.

## Patentansprüche

1. Abdeckmembran zum vorübergehenden Abdecken einer mit Knochenaufbaumaterial, wie Hydroxylapatitgranulat, gefüllten Ausnehmung in körpereigenem Knochengewebe welche aus resorbierbarem Membranmaterial (16) besteht,
**dadurch gekennzeichnet, daß** das Membranmaterial (16) von der dem körpereigenen Knochen (10) abgewandten Seite schneller resorbierbar ist als von der dem körpereigenen Knochen (10) zugewandten Seite, daß das Membranmaterial (16) aus mehreren Schichten (22, 24, 26, 28) unterschiedlicher Dichte und/oder Zusammensetzung besteht und daß das Membranmaterial (16) zumindest teilweise aus Kollagen besteht.

2. Abdeckmembran nach Anspruch 1, **dadurch gekennzeichnet, daß** die Dichte des Membranmaterials (16) von der dem körpereigenen Knochen (10) abgewandten Seite zu der dem körpereigenen Knochen (10) zugewandten Seite zunimmt.

3. Abdeckmembran nach Anspruch 1 oder 2, **dadurch gekennzeichnet, daß** das Membranmaterial (16) mehrere gewebte oder gewirkte Schichten (22, 24, 26, 28) unterschiedlicher Textur aufweist.

4. Abdeckmembran nach einem der vorangehenden Ansprüche, **dadurch gekennzeichnet, daß** das Membranmaterial (16) zumindest teilweise aus lyophilisierter Hirnhaut (dura mater) besteht.

5. Abdeckmembran nach einem der vorangehenden Ansprüche, **dadurch gekennzeichnet, daß** das Membranmaterial (16) zumindest teilweise aus Polyglactid/vicryl besteht.

6. Abdeckmembran nach einem der vorangehenden Ansprüche, **dadurch gekennzeichnet, daß** das Membranmaterial (16) zumindest teilweise aus Polylactid besteht.

7. Abdeckmembran nach einem der vorangehenden Ansprüche, **dadurch gekennzeichnet, daß** das Membranmaterial (16) zumindest teilweise aus Oxymethylcellulose besteht.

8. Abdeckmembran nach einem der vorangehenden Ansprüche, **gekennzeichnet durch** eine derartige Einstellung des von der dem körpereigenen Knochen (10) abgewandten Seite zu dem körpereigenen Knochen (10) zügewandten Seite abnehmenden Resorbierbarkeitsgradienten des Membranmaterials (16), daß die am Knochen (10) anliegende Schicht des Membranmaterials (16) erst dann von körpereigenem Gewebe resorbiert wird, wenn die **durch** die Abdeckmembran verschlossene Ausnehmung (12) des körpereigenen Knochens (10) im wesentlichen vollständig knöchern durchwachsen und in den Knochen (10) reintegriert ist.

9. Abdeckmembran nach einem der vorangehenden Ansprüche, **gekennzeichnet durch** eine Versteifungslage in Form eines perforierten Metallbleches oder dergleichen.

10. Abdeckmembran nach Anspruch 9, **dadurch gekennzeichnet, daß** das Metallblech aus Titan besteht.

## Claims

1. A covering membrane for temporarily covering a recess in endogenous bone tissue filled with bone-building material such as hydroxyl apatite granulate, the membrane being made of resorbable material (16), **characterised in that** the membrane material (16) is resorbable more quickly on the side remote from the endogenous bone (10) than on the side facing the endogenous bone (10), so that the membrane material (16) comprises a number of layers (22, 24, 26, 28) varying in density and/or composition and **in that** the membrane material (16) consists at least partly of collagen.

2. A covering membrane according to claim 1, **characterised in that** the density of the membrane material (16) increases from the side remote from the endogenous bone (10) to the side facing the endogenous bone (10).

3. A covering membrane according to claim 1 or 2, **characterised in that** the membrane material (16) comprises a number of woven or knitted layers (22, 24, 26, 28) varying in texture.

4. A covering membrane according to any of the preceding claims, **characterised in that** the membrane material (16) consists at least partly of freeze-dried meninges (dura mater).

5. A covering membrane according to any of the preceding claims, **characterised in that** the membrane material (16) consists at least partly of polyglactide/Vicryl.

6. A covering membrane according to any of the preceding claims, **characterised in that** the membrane material (16) consists at least partly of polylactide.

7. A covering membrane according to any of the preceding claims, **characterised in that** the membrane material (16) consists at least partly of oxymethyl cellulose.

8. A covering membrane according to any of the preceding claims, **characterised in that** the resorbability gradient of the membrane material (16) from the side remote from the endogenous bone (10) to the side facing the endogenous bone (10) is adjusted so that the layer of membrane material (16) adjoining the bone (10) is not resorbed by endogenous tissue until the recess (12) in the endogenous bone (10) closed by the membrane has been substantially completely overgrown by bony substance and is re-incorporated in the bone (10).

9. A covering membrane according to any of the preceding claims, **characterised by** a reinforcing layer in the form of a perforated metal plate or the like.

10. A covering membrane according to claim 9, **characterised in that** the metal plate is of titanium.

## Revendications

1. Membrane de recouvrement pour le recouvrement temporaire d'une cavité remplie d'un matériau de reconstitution osseuse, tel que du granulat d'hydroxylapatite, dans un tissu osseux propre au corps, formé d'un matériau de membrane (16) résorbable, **caractérisée en ce que** le matériau de membrane (16) est résorbable plus rapidement, depuis le côté opposé à l'os (10) propre au corps, que depuis le côté tourné vers l'os (10) propre au corps, **en ce que** le matériau de membrane (16) est formé de plusieurs couches (22, 24, 26, 28) de densité et/ou de composition différentes, et **en ce que** le matériau de membrane (16) est formé au moins partiellement de collagène.

2. Membrane de recouvrement selon la revendication 1, **caractérisée en ce que** la densité du matériau de membrane (16) va en augmentant, en allant du côté opposé à l'os (10) propre au corps vers le côté tourné vers l'os (10) propre au corps.

3. Membrane de recouvrement selon la revendication 1 ou 2, **caractérisée en ce que** le matériau de membrane (16) présente plusieurs couches (22, 24, 26, 28) tissées ou tressées, à textures différentes.

4. Membrane de recouvrement selon l'une des revendications précédentes, **caractérisée en ce que** le matériau de membrane (16) est formé au moins partiellement de méninge lyophilisée (dura mater).

5. Membrane de recouvrement selon l'une des revendications précédentes, **caractérisée en ce que** le matériau de membrane (16) est formé au moins partiellement de polylactide/vicryle.

6. Membrane de recouvrement selon l'une des revendications précédentes, **caractérisée en ce que** le matériau de membrane (16) est formé au moins partiellement de polylactide.

7. Membrane de recouvrement selon l'une des revendications précédentes, **caractérisée en ce que** le matériau de membrane (16) est formé au moins partiellement d'oxyméthylcellulose.

8. Membrane de recouvrement selon l'une des revendications précédentes, **caractérisée par** un réglage du gradient de résorbabilité du matériau de membrane (16), allant en diminuant, en allant du côté opposé à l'os (10) propre au corps vers le côté tourné vers l'os (10) propre au corps, tel que la couche, appliquée sur l'os (10), du matériau de membrane (16) est ensuite résorbée par le tissu propre au corps lorsque l'évidement (12), fermé par la membrane de recouvrement, de l'os (10) propre au corps, fait l'objet d'une croissance osseuse dans la masse pratiquement complète et réintégrée dans l'os (10).

9. Membrane de recouvrement selon l'une des revendications précédentes, **caractérisée par** une couche de rigidification ayant la forme d'une tôle métallique perforée ou analogue.

10. Membrane de recouvrement selon la revendication 9, **caractérisée en ce que** la tôle métallique est en titane.
